# FASCICULE DE BREVET EUROPEEN

(11) **EP 2 737 296 B1**
(45) Date de publication et mention de la délivrance du brevet: **29.04.2015**
(21) Numéro de dépôt: 12743521.2
(22) Date de dépôt: 17.07.2012
(51) Int. Cl.: G01N 3/10, G01N 33/10

(54) **EXTENSIMETRE POUR L'ANALYSE PAR SOUFFLAGE DES CARACTÉRISTIQUES MÉCANIQUES D'UN ÉCHANTILLON DE PÂTE À BASE DE FARINE DE CÉRÉALES**
EXTENSIOMETER ZUR BLASUNGSANALYSE DER MECHANISCHEN EIGENSCHAFTEN EINER MEHLTEIG PROBE
EXTENSIOMETER FOR THE BLOW ANALYSIS OF THE MECHANICAL CHARACTERISTICS OF A FLOUR PASTE SAMPLE

(30) Priorité: 25.07.2011 FR 1102318
(43) Date de publication de la demande: 04.06.2014
(73) Titulaire: Chopin Technologies, 92396 Villeneuve La Garenne Cedex (FR)
(72) Inventeur: HEFKA, Mathieu, F-92500 Rueil Malmaison (FR)
(74) Mandataire: Derambure Conseil
(86) Numéro de dépôt international: PCT/FR2012/051694
(87) Numéro de publication internationale: WO 2013/014366

(56) Documents cités:
- EP-A1- 0 879 555
- FR-A1- 2 913 772
- US-A- 5 280 717

## Description

La présente invention concerne un extensimètre perfectionné pour l'analyse par soufflage des caractéristiques mécaniques d'un échantillon de pâte à base de farine de céréale.

Il est connu d'analyser les caractéristiques d'un échantillon de pâte au moyen d'un extensimètre tel que celui connu sous la marque française « alvéographe » inventé par Monsieur CHOPIN et décrit notamment dans le document FR-525 686-A.

Cet extensimètre a fait l'objet de perfectionnements décrits dans le document FR-2 913 772-A permettant de souffler l'échantillon de pâte de telle sorte que la bulle se forme par extension vers le bas.

Si cet appareil donne satisfaction, par contre à l'usage, il est néanmoins apparu un certain nombre d'inconvénients en particulier pour la manipulation de l'échantillon de pâte ou pâton, ainsi qu'en ce qui concerne le maintien des conditions ambiantes (température et humidité notamment) autour de la bulle, afin d'assurer une meilleure répétitivité des mesures relatives aux caractéristiques mécaniques.

Parmi les inconvénients constatés, il a été relevé notamment ceux en liaison, d'une part, avec les moyens pour la mise en place du pâton et ceux pour la formation de la bulle, en particulier les moyens d'insufflation, et, d'autre part, avec le maintien notamment en température et humidité dans l'enceinte contenant l'extensimètre.

Aussi un des buts de la présente invention est-il de fournir un extensimètre perfectionné permettant d'obvier les inconvénients relevés.

Un autre but de l'invention est de fournir un tel extensimètre dont la mise en oeuvre est plus simple et permet une mesure plus fiable.

Ces buts, ainsi que d'autres qui apparaîtront par la suite, sont atteints par un extensimètre perfectionné pour l'analyse par soufflage des caractéristiques mécaniques d'un échantillon de pâte à base de céréale sous forme d'un disque, muni de moyens pour pincer le disque de pâte sur son pourtour, d'un moyen de mise sous pression d'un gaz pour former par soufflage une bulle et de moyens d'enregistrement de la pression exercée, et comportant un plateau supérieur fixe muni d'un orifice de soufflage obturable et une couronne qui détermine un ajour central et qui est disposée sous le plateau supérieur afin de pouvoir s'en rapprocher, être maintenue dans cette position, et respectivement s'en écarter, tandis qu'une platine support mobile est prévue pour transporter le disque de pâte sous la couronne, cette platine support mobile étant dimensionnée pour venir dans l'ajour central de la couronne et compléter celle-ci afin de former avec elle un plateau inférieur destiné à permettre l'écrasement du disque de pâte en se rapprochant du plateau supérieur, lequel extensimètre est caractérisé, selon la présente invention, par le fait qu'il comporte un dispositif pour retirer la pâte restant sur la couronne après éclatement de la bulle, comprenant une raclette de forme générale en fer à cheval, mobile autour d'un axe vertical situé sensiblement au sommet du fer à cheval et sur la couronne, et relié à un moteur.

De préférence, la raclette de forme générale en fer à cheval est animée d'un mouvement alternatif de rotation entre deux positions.

Avantageusement, l'angle formé par la raclette entre les deux positions est compris entre 45° et 90°, et est de préférence de l'ordre de 60°.

La description qui va suivre et qui ne présente aucun caractère limitatif, doit être lue en regard des figures annexées, parmi lesquelles :
- La figure 1 est une coupe verticale d'une vue en élévation d'un extensimètre selon la présente invention en position initiale avec trace de la bulle formée ;
- La figure 2 est une vue en perspective du seul dispositif pour retirer la pâte restant sur la platine après éclatement de la bulle ; et,
- La figure 3 représente en vue de dessus les différentes positions de la raclette lors de la mise en oeuvre du dispositif.

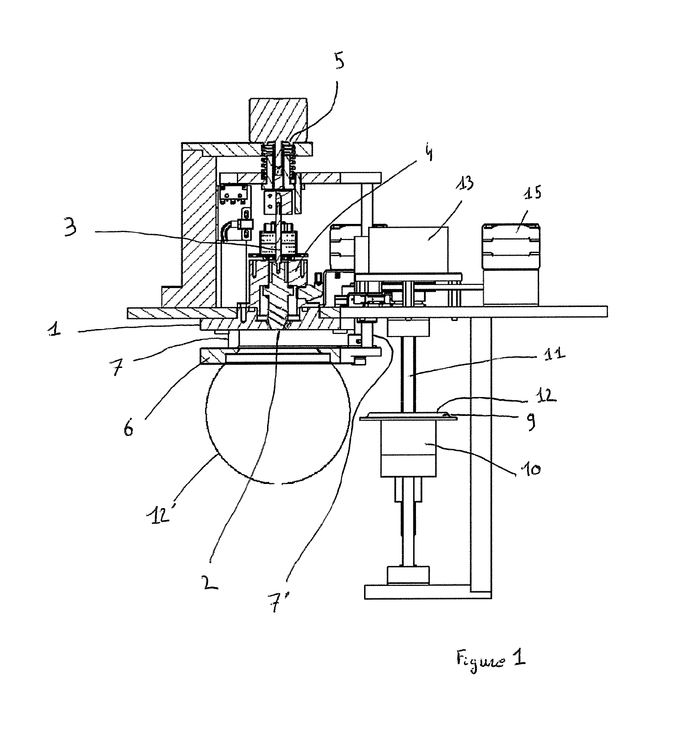

Ainsi qu'on peut le voir la figure 1, un extensimètre selon l'invention comporte essentiellement un plateau supérieur fixe 1 muni d'un orifice de soufflage 2 obturable, par exemple par une pointe d'obturation 3 montée dans un corps 4, tandis que des moyens d'alimentation en gaz, non représentés, sont prévus pour injecter du gaz tel que de l'air dans cet exemple, par les orifices de corps 4 visibles sur les dessins.

On comprend que la pointe 3 soumise à un moyen élastique peut être disposée au moyen par exemple d'un actionneur linéaire 5, soit dans une position dans laquelle l'orifice 2 est libre (figure 1), soit, au contraire, dans une position d'obturation partielle ou d'obturation totale.

L'extensimètre comporte une couronne 6 qui montée mobile en translation de manière à pouvoir se rapprocher par un ensemble d'actionneurs et réciproquement s'écarter du plateau supérieur 1.

Selon un mode de réalisation, chaque actionneur comporte, par exemple, une tige de guidage muni d'un ressort pour solliciter la couronne 6 à s'écarter et un moyen magnétique (par exemple une ventouse magnétique ou un vérin électrique) pour le maintien en position, mais on peut bien sûr utiliser d'autres types d'actionneurs.

La couronne 6 détermine un ajour central 8 en forme de disque.

Une platine support 9 est disposée à l'extrémité d'un bras 10 monté rotatif sur un arbre 11 (par exemple comme décrit dans le document FR-2 913 772-A).

La platine 9 sensiblement à l'horizontal est destinée à recevoir un échantillon de pâte sous la forme d'un disque de pâte ou pâton 12.

Un actionneur 13 (vérin électrique à came, électro-aimant,...) est associé à l'arbre 11 afin de mouvoir en translation le bras 10 et de ce fait la platine 9.

Par ailleurs, la platine 9 est dimensionnée de manière complémentaire et conjuguée de l'ajour 8 de façon à le boucher pour former avec la couronne 6 un plateau inférieur (6,9) complet. On peut également constater que, dans cet exemple et comme représenté, la platine 9 présente un épaulement correspondant à la forme conjuguée de l'ajour 8 de la couronne 6, assurant ainsi un guidage et un calage de la platine 9 dans l'ajour 8.

Comme expliqué dans le document FR-2 913 772-A, le disque de pâte ou pâton 12 est pincé à sa périphérie entre le plateau 1 et la couronne 6.

Le pâton 12, une fois coincé, est soumis à une insufflation continue de gaz de telle sorte qu'il forme une bulle 12' sous la couronne 6, laquelle bulle éclate. La couronne 6 est ensuite écartée du plateau 1. La partie centrale du pâton pend dans l'ajour 8 de la couronne 6 tandis que la collerette du pâton 12 adhère à la couronne 6 : le nettoyage de cette dernière partie pose problème, surtout si on ne veut pas modifier les conditions ambiantes présentes autour de la bulle.

Sur la couronne 6 est disposée, selon la présente invention, une raclette 16 en forme de fer à cheval qui entoure la périphérie du disque de pâte 12 une fois pincé entre le plateau 1 et la couronne 6. Cette raclette 16 est mobile autour d'un axe vertical 14 situé sensiblement au sommet du fer à cheval sur la couronne 6 et entraînée, de façon connue, dans un mouvement de rotation alternatif par un moteur 15.

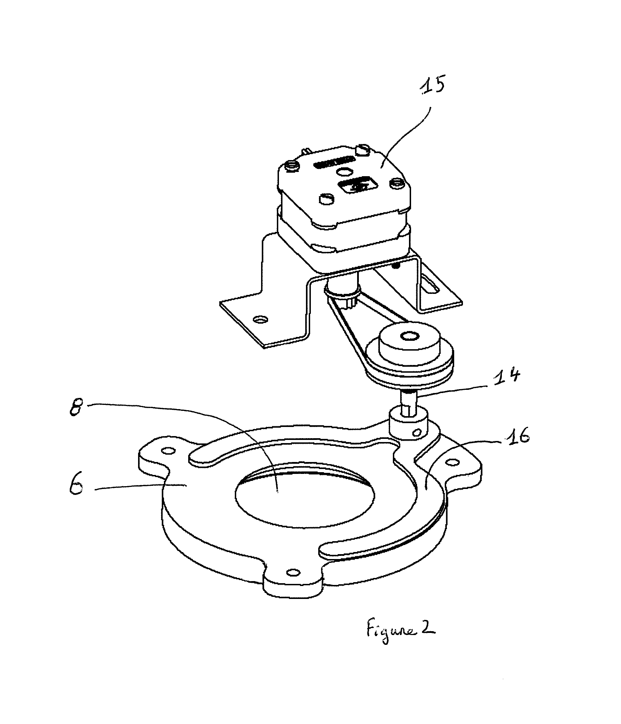

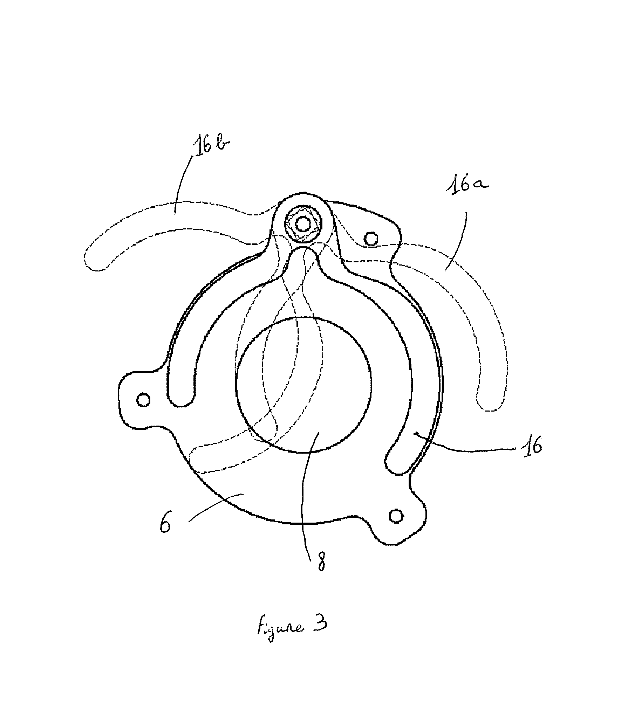

Comme présenté aux figures 2 et 3, l'éclatement de la bulle 12' ayant eu lieu, la raclette 16 est mise en mouvement par le moteur 15 et oscille entre deux positions, 16a et 16b, formant entre elles un angle compris entre 45° et 90° ; cet angle est de préférence de l'ordre d'une soixantaine de degrés. Ce mouvement alternatif de la raclette 16 permet ainsi de racler les morceaux de pâte restant sur la couronne 6 de telle sorte qu'aucun résidu de pâte ne subsiste sur la couronne 6, permettant ainsi un nouveau cycle de mesure.

Ainsi que l'aura compris l'homme du métier, ce dispositif comportant une raclette 16 entraînée en rotation alternative par un moteur 15 constitue un avantage important dans la mesure où il supprime toute intervention humaine, autre que celle de la mise en route du dispositif pour nettoyer la couronne après usage. De plus, ce dispositif permet de ne pas modifier les conditions ambiantes (en particulier la température et l'hygrométrie) autour de la bulle. Il pourrait même être envisagé d'asservir le moteur 15 à l'éjection de la bulle 12'.

## Revendications

1. Extensimètre perfectionné pour l'analyse par soufflage des caractéristiques mécaniques d'un échantillon de pâte à base de céréale sous forme d'un disque, muni de moyens pour pincer le disque de pâte sur son pourtour, d'un moyen de mise sous pression d'un gaz pour former par soufflage une bulle et de moyens d'enregistrement de la pressions exercée, et comportant un plateau supérieur (1) fixe muni d'un orifice de soufflage (2) obturable et une couronne (6) qui détermine un ajour (8) central et qui est disposé sous ledit plateau supérieur (1) afin de pouvoir s'en rapprocher, être maintenue dans cette position, et respectivement s'en écarter, tandis qu'une platine support mobile (9) est prévue pour transporter le disque de pâte sous ladite couronne (6), ladite platine support mobile (9) étant dimensionnée pour venir dans ledit ajour (8) central de ladite couronne (6) et compléter celle-ci afin de former avec elle un plateau inférieur destiné à permettre l'écrasement dudit disque de pâte en se rapprochant dudit plateau supérieur (1), **caractérisé par le fait qu'**il comporte un dispositif pour retirer la pâte restant sur ladite couronne (6) après éclatement de la bulle, comportant une raclette (16) de forme générale en fer à cheval, mobile autour d'un axe vertical situé sensiblement au sommet du fer à cheval et sur ladite couronne (6), et relié à un moteur.

2. Extensimètre selon la revendication 1, **caractérisé par le fait que** la raclette (16) de forme générale en fer à cheval est animée d'un mouvement alternatif de rotation entre deux positions (16a, 16b).

3. Extensimètre selon la revendication 2, **caractérisé par le fait que** l'angle formé par la raclette (16) entre les deux positions est compris entre 45° et 90°.

4. Extensimètre selon la revendication 2, **caractérisé par le fait que** l'angle formé par la raclette (16) entre les deux positions est de l'ordre de 60°.

5. Extensimètre selon l'une quelconque des revendications 1 à 4, dans lequel le dispositif pour retirer la pâte restant sur ladite couronne (6) après éclatement de la bulle est constitué par ladite raclette (16).

## Patentansprüche

1. Verbessertes Extensiometer zur Blasungsanalyse der mechanischen Eigenschaften einer Probe eines Teigs auf Getreidebasis in Form einer Scheibe, das mit Mitteln zum Einklemmen der Teigscheibe um ihren Umfang, einem Mittel zur Druckbeaufschlagung mit einem Gas zur Bildung einer Blase durch Blasen und Mitteln zur Aufzeichnung des ausgeübten Drucks versehen ist und eine feste obere Platte (1), die mit einer verschließbaren Blasöffnung (2) versehen ist, und einen Ring (6) umfasst, der einen mittleren Durchbruch (8) bestimmt und der unter der oberen Platte (1) angeordnet ist, um sich ihr annähern zu können, in dieser Position gehalten werden zu können, bzw. sich davon entfernen zu können, während ein beweglicher Träger (9) für die Beförderung der Teigscheibe unter den Ring (6) vorgesehen ist, wobei der bewegliche Träger (9) dazu dimensioniert ist, in den mittleren Durchbruch (8) des Rings (6) zu gelangen und diesen auszufüllen, um mit ihm eine untere Platte zu bilden, die das Zerdrücken der Teigscheibe durch Annähern an die obere Platte (1) gestattet, **dadurch gekennzeichnet, dass** es eine Vorrichtung zum Zurückziehen des auf dem Ring (6) verbleibenden Teigs nach Zerplatzen der Blase umfasst, die einen allgemein hufeisenförmigen Schaber (16) umfasst, der um eine sich im Wesentlichen am Scheitel des Hufeisens und auf dem Ring (6) befindende vertikale Achse beweglich ist und mit einem Motor verbunden ist.

2. Extensiometer nach Anspruch 1, **dadurch gekennzeichnet, dass** der allgemein hufeisenförmige Schaber (16) in eine hin- und hergehende Drehbewegung zwischen zwei Positionen (16a, 16b) versetzt wird.

3. Extensiometer nach Anspruch 2, **dadurch gekennzeichnet, dass** der durch den Schaber (16) zwischen den beiden Positionen gebildete Winkel zwischen 45° und 90° beträgt.

4. Extensiometer nach Anspruch 2, **dadurch gekennzeichnet, dass** der durch den Schaber (16) zwischen den beiden Positionen gebildete Winkel ca. 60° beträgt.

5. Extensiometer nach einem der Ansprüche 1 bis 4, wobei die Vorrichtung zum Zurückziehen des nach dem Zerplatzen der Blase auf dem Ring (6) verbleibenden Teigs durch den Schaber (16) gebildet wird.

## Claims

1. Improved extensimeter for analyzing by blowing the mechanical properties of a disk-shaped, grain-based dough sample, equipped with means for pinching the perimeter of the dough disk, a means for pressurizing a gas for blowing a bubble and means of recording the pressure exerted and comprising a fixed upper tray (1) equipped with a blowing orifice (2) which can be closed and a ring (6) which defines a central opening (8) and which is placed under said upper tray (1) in order to be able to get closer to it, being held in this position, and respectively move away from it, whereas a mobile support plate (9) is provided for transporting the dough disk under said ring (6), where said mobile support (9) is sized for coming into said central opening (8) of said ring (6) and supplementing it in order to form therewith a lower tray intended to allow crushing of said dough disk by moving closer to said upper tray (1), **characterized in that** it comprises a device for removing the dough which remains on said ring (6) after bursting of the bubble, comprising a generally horseshoe-shaped scraper (16), movable around a vertical axis located substantially at the summit of the horseshoe and on said ring (6), and connected to a motor.

2. Extensimeter according to claim 1, **characterized in that** generally horseshoe-shaped scraper (16) is driven in alternating rotational movement between two positions (16a, 16b).

3. Extensimeter according to claim 2, **characterized in that** the angle formed by the scraper 16 between both positions is included between 45° and 90°.

4. Extensimeter according to claim 2, **characterized in that** the angle formed by the scraper (16) between the two positions is of order 60°.

5. Extensimeter according to any one of claims 1 to 4, **characterized in that** the device for removing the dough remaining on said ring (6) after bursting of the bubble is made up of said scraper (16).
